(19) Europäisches Patentamt
European Patent Office
Office européen des brevets

(11) Numéro de publication: **0 239 500 B1**

# (12) FASCICULE DE BREVET EUROPEEN

(45) Date de publication de fascicule du brevet: **22.01.92**

(51) Int. Cl.[5]: **C07D 207/48**, A61K 31/40

(21) Numéro de dépôt: **87400689.3**

(22) Date de dépôt: **26.03.87**

Le dossier contient des informations techniques présentées postérieurement au dépôt de la demande et ne figurant pas dans le présent fascicule.

## (54) Dérivés de la 1-styrène sulfonyl 2-oxo 5-hydroxy pyrrolidine, leur procédé et des intermédiaires de préparation, leur application comme médicaments et les compositions les renfermant.

(30) Priorité: **28.03.86 IT 1991686**

(43) Date de publication de la demande: **30.09.87 Bulletin 87/40**

(45) Mention de la délivrance du brevet: **22.01.92 Bulletin 92/04**

(84) Etats contractants désignés: **AT BE CH DE ES FR GB GR IT LI LU NL SE**

(56) Documents cités:
**EP-A- 0 138 721**
**EP-A- 0 229 566**
**US-A- 3 453 289**
**US-A- 4 217 130**

(73) Titulaire: **ROUSSEL-UCLAF**
**35, Boulevard des Invalides**
**F-75007 Paris(FR)**

(72) Inventeur: **Toja, Emilio**
**Via Plezzo 80**
**I-20100 Milano(IT)**
Inventeur: **Zirotti, Carlo**
**Via 2 Giugno 12**
**I-Arona (NO)(IT)**
Inventeur: **Galliani, Giulio**
**Via U. Biancamano**
**I-20052 Monza (MI)(IT)**
Inventeur: **Barzaghi, Fernando**
**Via Monteverdi 21**
**I-20052 Monza (MI)(IT)**

(74) Mandataire: **Tonnellier, Marie-José et al**
**Département des Brevets ROUSSEL UCLAF**
**B.P. no 9**
**F-93230 Romainville(FR)**

**EP 0 239 500 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention concerne de nouveaux dérivés de la 1-styrène sulfonyl 2-oxo 5-hydroxy pyrrolidine, leur procédé et des intermédiaires de préparation, leur application comme médicaments et les compositions les renfermant.

La demande de brevet européen EP-A-138721 décrit des 4-alkyloxy ou 4-acyloxy 1-benzènesulfonyl 2-oxopyrrolidines qui peuvent être utilisés comme médicaments dans le traitement des troubles de la mémoire et dans le traitement de l'amnésie.

L'invention a pour objet les composés répondant à la formule générale (I) :

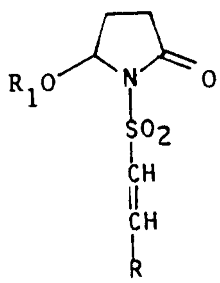

(I)

dans laquelle $R_1$ représente un radical alcoyle, linéaire ou ramifié, renfermant jusqu'à 8 atomes de carbone, un radical acyle renfermant de 1 à 6 atomes de carbone ou un radical cycloalcoyle renfermant de 3 à 6 atomes de carbone et R représente un radical phényle ou naphtyle, ou un radical furyle, thiényle, pyranyle, pyridyle, benzofuranyle, isobenzofuranyle, chromanyle, isochromanyle, chroményle, xanthényle, phénoxathiényle, oxazolyle, isoxazolyle, furazanyle, phénoxazinyle, thiéno[2,3-b] furanyle, 2H-furo[3,2-b]pyranyle, benzoxazolyle ou morpholinyle, R étant éventuellement substitué par un ou plusieurs substituants choisis dans le groupe constitué par les radicaux hydroxyles, acétoxy, méthoxy ou benzyloxy, par le radical

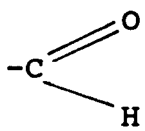 , 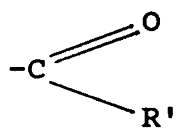 ou -C=NOR'

dans lesquels R' représente un radical alcoyle renfermant jusqu'à 8 atomes de carbone, par les radicaux méthyle, éthyle, propyle ou isopropyle, le radical éthényle ou le radical éthynyle, le fluor, le chlore, le brome, les groupements $SCF_3$, $OCF_3$, $NO_2$, $NH_2$ ou C≡N et le radical phényle.

La géométrie de la double liaison peut être trans ou cis. L'invention a pour objet les composés de formule (I) dans lesquels la géométrie de la double liaison est trans ou cis ainsi que les mélanges de ces produits de géométrie trans et cis.

Par radical alcoyle, on entend de préférence un radical alcoyle renfermant de 1 à 5 atomes de carbone, par exemple, le radical méthyle, éthyle, n-propyle, isopropyle, n-butyle, isobutyle, terbutyle ou n-pentyle.

Par radical acyle, on entend de préférence un radical acétyle, propionyle ou butyryle.

Par radical cycloalcoyle, on entend de préférence un radical cyclopropyle, cyclobutyle, cyclopentyle ou cyclohexyle.

L'invention a notamment pour objet les composés de formule (I) dans lesquels R représente un radical phényle éventuellement substitué par un radical alcoxy renfermant jusqu'à 4 atomes de carbone, comme par exemple le radical méthoxy.

L'invention a plus particulièrement pour objet les composés de formule (I) dans lesquels $R_1$ représente un radical éthyle.

L'invention a tout spécialement pour objet le produit de l'exemple 1.

L'invention a également plus particulièrement pour objet les produits décrits dans les exemples 2 et suivants.

Les composés de l'invention présentent d'intéressantes propriétés pharmacologiques ; ils retardent l'extinction de la réponse d'évitement conditionné, ils retardent la disparition de la réponse apprise. Ils favorisent l'attention, la vigilance et la mémorisation.

L'invention a donc pour objet les produits de formule (I) en tant que médicament, utiles notamment

dans le traitement des asthénies intellectuelles ou nerveuses, les défaillances de la mémoire, la sénescence, le surmenage intellectuel.

L'invention a plus particulièrement pour objet en tant que médicament le produit de l'exemple 1.

La posologie usuelle est variable selon l'affection en cause, le sujet traité et la voie d'administration, elle peut être comprise entre 50 mg et 3000 mg/jour, par exemple entre 150 et 1500 mg/jour en une ou plusieurs prises pour le produit de l'exemple 1 administré par voie orale.

La présente invention a également pour objet les compositions pharmaceutiques renfermant comme principe actif au moins un produit de formule (I). Les compositions pharmaceutiques de l'invention peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables ; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animales ou végétale, les dérivés paraffiniques, les glycols, les divers agents mouillants, dispersants ou émulsifiants, les conservateurs.

L'invention a également pour objet un procédé de préparation des composés de formule (I) caractérisé en ce que l'on soumet un composé de formule (II) :

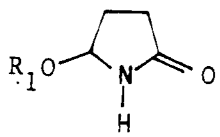

(II)

dans laquelle $R_1$ conserve la même signification que précédemment, à l'action d'un composé de formule (III) :

$RCH=CH\text{-}SO_2Hal$ (III)

dans laquelle Hal représente un atome de chlore ou de brome et R conserve la même signification que précédemment, pour obtenir le composé de formule (I) correspondant.

Dans un mode de réalisation préférée du procédé de l'invention, la réaction entre le produit de formule (II) et le produit de formule (III) est effectuée :

a) en présence d'une base forte comme le butyllithium ou un hydrure alcalin, comme l'hydrure de sodium,

b) au sein d'un solvant choisi dans le groupe constitué par le tétrahydrofuranne, le benzène, le diméthylformamide, le diméthylsulfoxyde, l'éther monoéthylique du diéthylène glycol, ou l'éther diéthylique du diéthylène glycol.

Les produits de formule (II) utilisés comme produits de départ sont des produits connus de façon générale et peuvent être préparés selon les procédés décrits dans Tétrahedron 31, 1437 (1975), Tétrahedron 41, 2007 (1985) ou dans Synthesis (4) 315-17 (1980).

Les produits de formule (III), utilisés comme produits de départ sont connus de façon générale : ils peuvent être préparés selon le procédé décrit dans CA 47 3262 c (1953).

Le chlorure de 4-méthoxy styrène sulfonyle est toutefois un produit nouveau et est donc à ce titre, un objet de la présente invention.

Les exemples suivants illustrent l'invention sans toutefois la limiter.

**Exemple 1 : 1-(styrène sulfonyl) 2-oxo 5-éthoxy pyrrolidine.**

On dissout 2,6 g de 2-oxo 5-éthoxy pyrrolidine dans 80 $cm^3$ de tétrahydrofuranne anhydre, refroidit à -5 °C, ajoute 12,6 $cm^3$ de butyllithium. Après 20 minutes sous agitation, on ajoute à la même température lentement, 4,05 g de chlorure de styrène sulfonyl (CA 47, 3262c (1953) dans 10 $cm^3$ de tétrahydrofuranne. On laisse revenir à température ambiante, après 4 heures, on concentre à sec et chromatographie le résidu sur silice. On obtient 1,3 g de produit attendu que l'on cristallise dans un mélange éther éthylique-hexane. F = 61-63 °C.

Analyse : $C_{14}H_{17}NO_4S$

Calculé : C% 56,93 H% 5,8 N% 4,74

Trouvé : 56,85 5,63 4,57

**Exemple 2 : 1-(4-méthoxy styrène sulfonyl) 2-oxo 5-éthoxy pyrrolidine.**

On dissout 2,2 g de 2-oxo 5-éthoxy pyrrolidine dans 170 $cm^3$ de tétrahydrofuranne anhydre puis ajoute sous gaz inerte à -10, -15 °C, 10,6 cm3 d'une solution 1,6 M de butyllithium dans l'hexane. Après 30 minutes, on ajoute lentement 4,1 g de chlorure de 4-méthoxy styrène sulfonyl, laisse revenir lentement à température ambiante et concentre à sec. On chromatographie le résidu sur silice et obtient 1,5 g de produit que l'on recristallise dans l'isopropanol. On obtient 1,15 g de produit attendu. F = 134-136¤C.
Analyse : $C_{15}H_{19}NO_5S$
Calculé : C% 55,36 H% 5,89 N% 4,30
Trouvé : 55,24 H% 5,83 N% 4,16

**Préparation du chlorure de 4-méthoxy styrène sulfonyl.**

On chauffe à 110¤C pendant 9 heures 30 minutes en enceinte close un mélange de 5,5 g de paraméthoxy styrène, 13 g de $SO_3$-pyridine et 5 cm3 de dichloroéthane. On refroidit, dilue dans le chlorure de méthylène puis concentre à sec. On ajoute au mélange 7,6 g de pentachlorure de phosphore et chauffe aux environs de 85¤C jusqu'à dissolution totale puis pendant 3 heures à 45¤C. On concentre à sec, verse dans un mélange eau-glace, extrait au chloroforme, sèche et concentre à sec. On chromatographie rapidement le mélange sur silice en diluant au mélange hexane-chloroforme (1-1). On obtient un produit (65-67¤C) puis on cristallise dans le mélange hexane-chloroforme en refroidissant pendant 40 minutes. On obtient 3,4 g de produit attendu. F = 73-74¤C.

**Exemple 3 : 1-(styrène sulfonyl) 2-oxo 5-méthoxy pyrrolidine.**

On dissout 3,29 g de 5-méthoxy 2-oxo pyrrolidine (Synthesis 4, 315-17 (1980) dans 100 cm3 de tétrahydrofuranne anhydre. On refroidit à -20¤C puis ajoute 17,86 cm3 d'une solution 1,6 M de n-butyllithium dans l'hexane en maintenant la température aux environs de -30¤C. On agite pendant 30 minutes à -35,-30¤C puis ajoute lentement une solution de 5,80 g de chlorure de styrène sulfonyl (CA 47, 3262c, 1953) dans 14 cm3 de tétrahydrofuranne anhydre. On laisse revenir à température ambiante, évapore le solvant sous pression réduite et chromatographie le résidu sur gel de silice en éluant au mélange acétate d'éthyle- n-hexane (1-1). On cristallise le produit obtenu dans l'isopropanol et obtient 3 g de produit attendu. F = 98-99¤C.
Analyse : $C_{13}H_{15}NO_4S$
Calculé : C% 55,50 H% 5,37 N% 4,98
Trouvé : 55,22 H% 5,17 N% 4,89

**Exemple 4 : 1-(styrène sulfonyl) 2-oxo 5-n-propoxy pyrrolidine.**

A une solution de 4,09 g de 2-oxo 5-n-propyloxy pyrrolidine dans 100 cm3 de tétrahydrofuranne anhydre, refroidit à -30¤C, on ajoute 17,89 cm3 d'une solution 1,6 M de n-butyllithium dans l'hexane en opérant à -30,-25¤C. On agite pendant 30 minutes à -30¤C, ajoute lentement une solution de 5,8 g de chlorure de styrène sulfonyl dans 14 cm3 de tétrahydrofuranne anhydre (CA 47, 3262c, 1953). On laisse revenir à température ambiante, évapore le solvant sous pression réduite et chromatographie le résidu sur gel de silice en éluant au mélange acétate d'éthyle-n-hexane (1-1). On cristallise le résidu dans l'isopropanol et obtient 3 g de produit attendu. F = 74-75¤C.
Analyse : $C_{15}H_{19}NO_4S$ (309,40)
Calculé : C% 58,23 H% 6,19 N% 4,53
Trouvé : 58,35 H% 6,18 N% 4,49

**Préparation du 2-oxo 5-n-propyloxy pyrrolidine.**

A un mélange de 28,64 g de succinimide de 1200 cm3 de n-propanol, on refroidit à -7¤C, on ajoute 7 g de borohydrure de sodium. On agite pendant 4 heures à -7,0¤C en ajoutant toutes les 15 minutes 15 gouttes d'une solution de 2 N d'acide chlorhydrique dans le n-propanol. On porte ensuite à pH environ 2 en ajoutant une solution 2N d'acide chlorhydrique dans le n-propanol, agite pendant 1 heure à environ 0¤C, revient à pH 7 par addition d'une solution saturée d'hydroxyde de potassium dans le n-propanol. On laisse

revenir à température ambiante puis évapore le solvant sous pression réduite. On extrait le résidu par 550 cm3 de chloroforme, filtre, lave la solution organique à l'eau, sèche et évapore le solvant sous pression réduite. On obtient 27,5 g de produit attendu. F = 52-54¤C.

**Exemple 5 : 1-(styrène sulfonyl) 2-oxo 5-isopropyloxy pyrrolidine.**

A une solution de 3,53 g de 2-oxo 5-isopropyloxy pyrrolidine dans 85 cm3 de tétrahydrofuranne anhydre refroidie à -10¤C, on ajoute 15,42 g d'une solution 1,6 M de n-butyllithium dans l'hexane en opérant à -10,-5¤C. On agite pendant 30 minutes en refroidissant à -45¤C, ajoute lentement une solution de 5 g de chlorure de styrène sulfonyl (CA 47, 3262c, 1953) dans 35 cm3 de tétrahydrofuranne anhydre en opérant à -45,-40¤C. On laisse revenir à température ambiante, évapore le solvant sous pression réduite et chromatographie le résidu sur silice en éluant au mélange acétate d'éthyle-hexane (1-1). On cristallise le produit obtenu dans l'éthanol et obtient 3,1 g de produit attendu. F = 92-94¤C.
Analyse : $C_{15}H_{19}NO_4S$
Calculé : C% 58,23 H% 6,19 N% 4,53
Trouvé : 58,04 H% 6,07 N% 4,42

**Préparation du 2-oxo 5-isopropyloxy pyrrolidine.**

A un mélange de 28,64 g de succinimide dans 1200 cm3 d'isopropanol refroidie à -10¤C, on ajoute 32,8 g de borohydrure de sodium et agite pendant 4 heures en maintenant la température à -10,0¤C. On ajoute toutes les 15 minutes 15 gouttes d'une solution 2N d'acide chlorhydrique dans l'isopropanol. En opérant à 0,+2¤C, on porte ensuite le mélange réactionnel à pH 2 - 3 par addition d'une solution 2N d'acide chlorhydrique dans l'isopropanol puis agite pendant 2 heures à la même température. On neutralise ensuite par addition d'une solution saturée hydroxyde de potassium dans l'isopropanol, en opérant à 0¤C. On évapore l'isopropanol sous pression réduite, extrait au chloroforme, filtre et évapore le solvant sous pression réduite. On obtient 20,5 g de produit attendu. F = 68-71¤C.

**Exemple 6 : 1-(4-nitro styrène sulfonyl) 2-oxo 5-éthoxy pyrrolidine.**

On dissout 3,25 g de 5-éthoxy pyrrolidine 2-one dans 100 cm3 de tétrahydrofuranne anhydre et ajoute à-25¤C, sous agitation, 16,25 cm3 de butyllithium à 15% dans l'hexane. Après 15 minutes sous agitation, on ajoute à -30¤C, 6,2 g de chlorure de béta-4-nitro styrène sulfonyle (J. Am. Chem. Soc. 68, 1778, 1946) dissous dans 100 cm3 de tétrahydrofuranne. Après 2 heures, on laisse revenir à température ambiante, concentre à sec, ajoute de l'eau et fait cristalliser. On filtre les cristaux, les sèche et chromatographie sur silice en éluant au mélange toluène-acétate d'éthyle (8-2). On obtient 2,7 g de produit attendu que l'on cristallise dans l'isopropanol. On obtient 2,4 g de produit. F = 162-163¤C.
Analyse : $C_{14}H_{16}N_2O_6S$ : 340,36.
Calculé : C% 49,40 H% 4,74 N% 8,23
Trouvé : 48,93 H% 4,72 N% 8,08

**Exemple 7 : 1-(4-méthoxy styrène sulfonyl) 2-oxo 5-isopropoxy pyrrolidine.**

On dissout 2,89 g de 5-isopropoxy pyrrolidine 2-one dans 100 cm3 de tétrahydrofuranne anhydre puis ajoute sous gaz inerte à -30¤C,-32¤C, 13,4 cm3 d'une solution 1,6 M de butyllithium dans l'hexane. Après 25 minutes à -40¤C, on ajoute lentement 4,7 g de chlorure de béta-4-méthoxy styrène sulfonyle en solution dans 15 cm3 de tétrahydrofuranne. On laisse revenir lentement à température ambiante, concentre à sec et chromatographie sur silice en éluant au mélange toluène-acétate d'éthyle (8-2). On recristallise le produit dans l'éther isopropylique et obtient 2,4 g de produit attendu. F = 101-103¤C.
Analyse : $C_{16}H_{21}NO_5S$ : 339,41
Calculé : C% 56,62 H% 6,23 N% 4,12
Trouvé : 56,83 H% 6,31 N% 4,21

**Exemple 8 : 1-(4-nitro styrène sulfonyl) 2-oxo 5-isopropoxy pyrrolidine.**

On dissout 3,46 g de 5-isopropoxy pyrrolidine 2-one dans 100 cm3 de tétrahydrofuranne anhydre et ajoute sous agitation à -30¤C, 16 cm3 de butyllithium à 15% dans l'hexane. Après 20 minutes, on ajoute à -35¤C, 6 g de chlorure de béta-4-nitro styrène sulfonyle en solution dans 100 cm3 de tétrahydrofuranne

anhydre. Après 2 heures, on laisse revenir à température ambiante, concentre et fait cristalliser dans l'isopropanol. On filtre les cristaux et les recristallise dans l'isopropanol. On obtient 3 g de produit attendu. F = 178-179¤C.

Analyse : $C_{15}H_{18}N_2O_6S$ : 354,396
Calculé : C% 50,84 H% 5,12 N% 7,90
Trouvé : 50,66 H% 5,24 N% 7,87

**Exemple 9 : 1-(styrène sulfonyl) 2-oxo 5-pentyloxy pyrrolidine.**

On dissout 2 g de 5-pentyloxy pyrrolidine 2-one dans 90 cm3 de tétrahydrofuranne anhydre. On refroidit à -30¤C sous agitation et sous gaz inerte et ajoute 7,7 cm3 d'une solution 1,5 M de butyllithium dans l'hexane. Après 25 minutes, on ajoute lentement, à -40¤C, 2,36 g de chlorure de béta-styrène sulfonyle en solution dans le tétrahydrofuranne. On laisse revenir lentement à température ambiante, concentre à sec, chromatographie sur silice en éluant au mélange toluène-acétate d'éthyle (8-2), recristallise dans l'éther isopropylique et obtient 2,1 g de produit attendu. F = 66-68¤C.

Analyse : $C_{17}H_{25}NO_4S$ : 337,44
Calculé : C% 60,51 H% 6,87 N% 4,15
Trouvé : 60,46 H% 6,79 N% 4,28

En utilisant une méthode analogue à celle utilisée dans les exemples 1 à 9, on peut notamment préparer les produits suivants :

- la 1-(4-phényl styrène sulfonyl) 2-oxo 5-éthoxy pyrrolidine, F = 132-134¤C,
- la 1-(4-phényl styrène sulfonyl) 2-oxo 5-isopropoxy pyrrolidine, F = 137-139¤C.

**ETUDE PHARMACOLOGIQUE**

**Toxicité aiguë et comportement.**

Nous avons utilisé des souris mâles ($CD_1$ Charles Rivers) d'un poids de 22-23 g à jeun depuis 16 heures. Les produits leur furent administrés normalement par voie orale aux doses de 1000 - 500 - 250 mg/kg.

L'effet des produits sur le comportement des animaux fut évalué suivant la méthode décrite par Irvin (Psychopharmacologia 13, 222-257, 1968) durant les 8 premières heures et à la 24ème heure.

La mortalité fut relevée pendant les 7 jours suivant le traitement.

La $DL_{50}$ a ainsi été trouvée supérieure à 1000 mg/kg sur les produits des exemples 1 à 9.

**Apprentissage et mémorisation.**

Nous avons utilisé des souris mâles ($CD_1$ Charles Rivers) d'un poids de 25-30 g. Les animaux sont placés dans la partie lumineuse d'un box à deux compartiments communicants par une ouverture (F. Barzaghi et G. Giuliani, Brit. J. Pharmacol. 86, 661 P. 1985).

A l'instant où la souris passe du compartiment lumineux au compartiment obscur, l'ouverture se ferme et elle est immédiatement punie par une décharge électrique aux pattes. L'animal soumis à cette procédure apprend à mémoriser la punition. En fait, si on le remet dans le compartiment lumineux, il évitera ainsi de franchir l'ouverture et de rentrer dans le compartiment obscur.

Pour induire une amnésie rétrograde, les animaux sont soumis immédiatement après l'apprentissage à un électrochoc. Après l'électrochoc, les produits sont administrés par voie orale aux doses de 12,5; 25; 50; 100 et 200 mg/kg.

Nous avons utilisé de 10 à 30 animaux par dose.

L'effet antiamnésique des produits est évalué 3 heures après le traitement, en utilisant le même protocole que celui utilisé pour l'acquisition.

Le temps mis par les animaux pour retourner dans le compartiment obscur (temps limite 180 secondes) est utilisé comme paramètre d'évaluation.

Dans nos conditions expérimentales, les animaux témoins entrèrent avec un temps de latence de 40-50 secondes.

Les résultats sont exprimés en pourcentage d'augmentation du temps de latence par rapport aux témoins correspondants.

Les résultats sont les suivants :

| Pourcentage d'augmentation du temps de latence par rapport aux témoins | | | | | |
|---|---|---|---|---|---|
| Produit de l'exemple | DOSE : mg/kg : os | | | | |
| | 200 | 100 | 50 | 25 | 12,5 |
| 1 | +108* | +110* | + 91* | +50* | +19* |
| 2 | + 50* | + 20 | + 14 | +14 | - |
| 3 | +102* | + 47 | + 12 | +21 | - |
| 4 | +103* | +103* | + 50* | +29 | - |
| 5 | +130* | + 98* | + 66* | +22 | - |
| 7 | + 68* | + 40* | - | - | - |
| 8 | + 49* | + 53* | + 29 | +23 | - |
| 9 | + 57* | + 60* | + 39 | +27 | + 9 |
| Piracétam | + 20 | + 48* | + 10 | +19 | - |
| Aniracétam | + 32 | + 88* | + 77* | +39 | - |

* Valeurs notablement différentes par rapport aux témoins.

Les produits des exemples 1 à 5 et 7 à 9 se sont montrés actifs. En particulier, le produit de l'exemple 1 améliore de manière significative le comportement des animaux à une dose 2 fois inférieure à celle de l'aniracétam. En outre, la gamme de doses efficaces est plus large que dans le cas de l'aniracétam.

**Exemples de compositions pharmaceutiques.**

a) On a préparé des comprimés répondant à la formule suivante :

| | |
|---|---|
| - Produit de l'exemple 1 | 100 mg |
| - Excipient q.s. pour un comprimé terminé à | 300 mg. |

(Détail de l'excipient : lactose, amidon de blé, amidon traité, amidon de riz, stéarate de magnésium, talc).

b) On a préparé des gélules répondant à la formule suivante :

| | |
|---|---|
| - Produit de l'exemple 1 | 200 mg |
| - Excipient q.s. pour un gélule terminée à | 300 mg. |

(Détail de l'excipient : talc, stéarate de magnésium, aérosil).

**Revendications**

**Revendications pour les Etats contractants suivants : BE, CH, DE, FR, GB, IT, LI, LU, NL, SE**

**1.** Les composés répondant à la formule générale (I) :

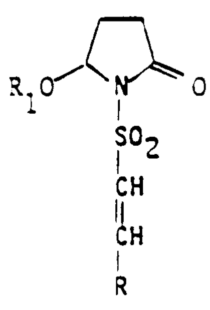

(I)

dans laquelle $R_1$ représente un radical alcoyle, linéaire ou ramifié, renfermant jusqu'à 8 atomes de carbone, un radical acyle renfermant de 1 à 6 atomes de carbone ou un radical cycloalcoyle

renfermant de 3 à 6 atomes de carbone et R représente un radical phényle ou naphtyle, ou un radical furyle, thiényle, pyranyle, pyridyle, benzofuranyle, isobenzofuranyle, chromanyle, isochromanyle, chroményle, xanthényle, phénoxathiényle, oxazolyle, isoxazolyle, furazanyle, phénoxazinyle, thiéno[2,3-b] furanyle, 2H-furo[3,2-b]pyranyle, benzoxazolyle ou morpholinyle, R étant éventuellement substitué par un ou plusieurs substituants choisis dans le groupe constitué par les radicaux hydroxyles, acétoxy, méthoxy ou benzyloxy, par le radical

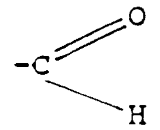 , 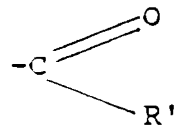 ou -C=NOR'

dans lesquels R' représente un radical alcoyle renfermant jusqu'à 8 atomes de carbone, par les radicaux méthyle, éthyle, propyle ou isopropyle, le radical éthényle ou le radical éthynyle, le fluor, le chlore, le brome, les groupements $SCF_3$, $OCF_3$, $NO_2$, $NH_2$ ou C≡N et le radical phényle.

**2.** Les composés de formule (I) tels que définis à la revendication 1, dans lesquels R représente un radical phényle éventuellement substitué par un radical alcoxy renfermant jusqu'à 4 atomes de carbone.

**3.** Les composés tels que définis à la revendication 1 ou 2, dans lesquels $R_1$ représente un radical éthyle.

**4.** Le composé tel que défini à la revendication 3 dont le nom suit :
- la 1-(styrène sulfonyl) 2-oxo 5-éthoxy pyrrolidine.

**5.** A titre de médicaments, les composés tels que définis à l'une quelconque des revendications 1 à 3.

**6.** A titre de médicament, le composé défini à la revendication 4.

**7.** Les compositions pharmaceutiques renfermant comme principe actif au moins un médicament défini à la revendication 5 ou 6.

**8.** Procédé de préparation des composés de formule (I) tels que définis à l'une quelconque des revendications 1 à 4, caractérisé en ce que l'on soumet un composé de formule (II) :

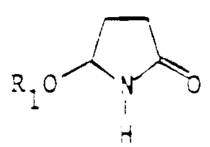

(II)

dans laquelle $R_1$ conserve la même signification que précédemment, à l'action d'un composé de formule (III) :

$RCH=CH\text{-}SO_2Hal$ (III)

dans laquelle Hal représente un atome de chlore ou de brome et R conserve la même signification que dans la revendication 1, pour obtenir le composé de formule (I) correspondant.

**9.** A titre de produit intermédiaire nécessaire à la mise en oeuvre du procédé de la revendication 8 :
- le chlorure de 4-méthoxy styrène sulfonyle.

**Revendications pour l'Etat contractant suivant : AT**

**1.** Procédé pour préparer des composés répondant à la formule générale (I) :

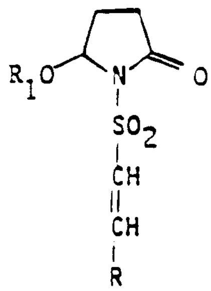

(I)

dans laquelle $R_1$ représente un radical alcoyle, linéaire ou ramifié, renfermant jusqu'à 8 atomes de carbone, un radical acyle renfermant de 1 à 6 atomes de carbone ou un radical cycloalcoyle renfermant de 3 à 6 atomes de carbone et R représente un radical phényle ou naphtyle, ou un radical furyle, thiényle pyranyle, pyridyle, benzofuranyle, isobenzofuranyle, chromanyle, isochromanyle, chroményle, xanthényle, phénoxathiényle, oxazolyle, isoxazolyle, furazanyle, phénoxazinyle, thiéno[2,3-b] furanyle, 2H-furo[3,2-b]pyranyle, benzoxazolyle ou morpholinyle, R étant éventuellement substitué par un ou plusieurs substituants choisis dans le groupe constitué par les radicaux hydroxyles, acétoxy, méthoxy ou benzyloxy, par le radical

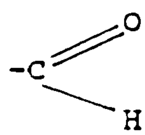 , 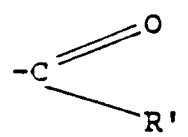 ou -C=NOR'

dans lesquels R' représente un radical alcoyle renfermant jusqu'à 8 atomes de carbone, par les radicaux méthyle, éthyle, propyle ou isopropyle, le radical éthényle ou le radical éthynyle, le fluor, le chlore, le brome, les groupements $SCF_3$, $OCF_3$, $NO_2$, $NH_2$ ou C≡N et le radical phényle, caractérisé en ce que l'on soumet un composé de formule (II) :

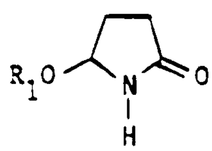

(II)

dans laquelle $R_1$ conserve la même signification que précédemment, à l'action d'un composé de formule (III) :

$RCH=CH\text{-}SO_2Hal$ (III)

dans laquelle Hal représente un atome de chlore ou de brome et R conserve la même signification que dans la revendication 1, pour obtenir le composé de formule (I) correspondant.

**2.** Procédé selon la revendication 1, caractérisé en ce que l'on utilise au départ un composé de formule (III) dans laquelle R représente un radical phényle éventuellement substitué par un radical alcoxy renfermant jusqu'à 4 atomes de carbone.

**3.** Procédé selon la revendication 1 ou 2, caractérisé en ce que l'on utilise au départ un composé de formule (II) dans laquelle $R_1$ représente un radical éthyle.

**4.** Procédé selon la revendication 1, 2 ou 3, caractérisé en ce que l'on utilise au départ un composé de formule (III) dans laquelle R représente un radical phényle et un composé de formule (II) dans laquelle $R_1$ représente un radical éthyle.

**Revendications pour l'Etat contractant suivant : ES**

1. Procédé pour préparer des composés répondant à la formule générale (I) :

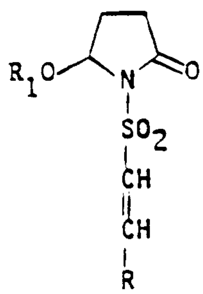

(I)

dans laquelle $R_1$ représente un radical alcoyle, linéaire ou ramifié, renfermant jusqu'à 8 atomes de carbone, un radical acyle renfermant de 1 à 6 atomes de carbone ou un radical cycloalcoyle renfermant de 3 à 6 atomes de carbone et R représente un radical phényle ou naphtyle, ou un radical furyle, thiényle, pyranyle, pyridyle, benzofuranyle, isobenzofuranyle, chromanyle, isochromanyle, chroményle, xanthényle, phénoxathiényle, oxazolyle, isoxazolyle, furazanyle, phénoxazinyle, thiéno[2,3-b]furanyle, 2H-furo[3,2-b]pyranyle, benzoxazolyle ou morpholinyle, R étant éventuellement substitué par un ou plusieurs substituants choisis dans le groupe constitué par les radicaux hydroxyles, acétoxy, méthoxy ou benzyloxy, par le radical

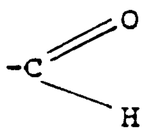 , 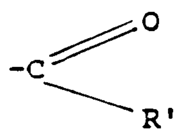 ou $-C=NOR'$

dans lesquels R' représente un radical alcoyle renfermant jusqu'à 8 atomes de carbone, par les radicaux méthyle, éthyle, propyle ou isopropyle, le radical éthényle ou le radical éthynyle, le fluor, le chlore, le brome, les groupements $SCF_3$, $OCF_3$, $NO_2$, $NH_2$ ou $C{\equiv}N$ et le radical phényle, caractérisé en ce que l'on soumet un composé de formule (II) :

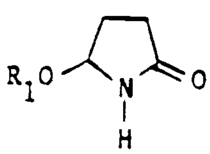

(II)

dans laquelle $R_1$ conserve la même signification que précédemment, à l'action d'un composé de formule (III) :

$RCH=CH\text{-}SO_2Hal$ (III)

dans laquelle Hal représente un atome de chlore ou de brome et R conserve la même signification que dans la revendication 1, pour obtenir le composé de formule (I) correspondant.

2. Procédé selon la revendication 1, caractérisé en ce que l'on utilise au départ un composé de formule (III) dans laquelle R représente un radical phényle éventuellement substitué par un radical alcoxy renfermant jusqu'à 4 atomes de carbone.

3. Procédé selon la revendication 1 ou 2, caractérisé en ce que l'on utilise au départ un composé de formule (II) dans laquelle $R_1$ représente un radical éthyle.

4. Procédé selon la revendication 1, 2 ou 3, caractérisé en ce que l'on utilise au départ un composé de formule (III) dans laquelle R représente un radical phényle et un composé de formule (II) dans laquelle $R_1$ représente un radical éthyle.

**Revendications pour l'Etat contractant suivant : GR**

1. Procédé pour préparer des composés répondant à la formule générale (I) :

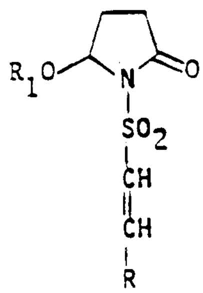

(I)

dans laquelle $R_1$ représente un radical alcoyle, linéaire ou ramifié, renfermant jusqu'à 8 atomes de carbone, un radical acyle renfermant de 1 à 6 atomes de carbone ou un radical cycloalcoyle renfermant de 3 à 6 atomes de carbone et R représente un radical phényle ou naphtyle, ou un radical furyle, thiényle, pyranyle, pyridyle, benzofuranyle, isobenzofuranyle, chromanyle, isochromanyle, chroményle, xanthényle, phénoxathiényle, oxazolyle, isoxazolyle, furazanyle, phénoxazinyle, thiéno[2,3-b] furanyle, 2H-furo[3,2-b]pyranyle, benzoxazolyle ou morpholinyle, R étant éventuellement substitué par un ou plusieurs substituants choisis dans le groupe constitué par les radicaux hydroxyles, acétoxy, méthoxy ou benzyloxy, par le radical

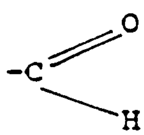 , 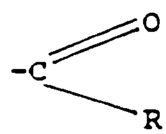 ou -C=NOR'

dans lesquels R' représente un radical alcoyle renfermant jusqu'à 8 atomes de carbone, par les radicaux méthyle, éthyle, propyle ou isopropyle, le radical éthényle ou le radical éthynyle, le fluor, le chlore, le brome, les groupements $SCF_3$, $OCF_3$, $NO_2$, $NH_2$ ou C≡N et le radical phényle, caractérisé en ce que l'on soumet un composé de formule (II) :

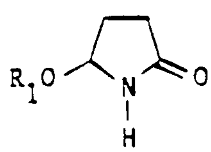

(II)

dans laquelle $R_1$ conserve la même signification que précédemment, à l'action d'un composé de formule (III) :

$RCH=CH\text{-}SO_2Hal$ (III)

dans laquelle Hal représente un atome de chlore ou de brome et R conserve la même signification que dans la revendication 1, pour obtenir le composé de formule (I) correspondant.

2. Procédé selon la revendication 1, caractérisé en ce que l'on utilise au départ un composé de formule (III) dans laquelle R représente un radical phényle éventuellement substitué par un radical alcoxy

renfermant jusqu'à 4 atomes de carbone.

**3.** Procédé selon la revendication 1 ou 2, caractérisé en ce que l'on utilise au départ un composé de formule (II) dans laquelle $R_1$ représente un radical éthyle.

**4.** Procédé selon la revendication 1, 2 ou 3, caractérisé en ce que l'on utilise au départ un composé de formule (III) dans laquelle R représente un radical phényle et un composé de formule (II) dans laquelle $R_1$ représente un radical éthyle.

**5.** A titre de produit intermédiaire nécessaire a la mise en oeuvre du procédé de la revendication 1 :
- le chlorure de 4-méthoxy styrène sulfonyle.

**6.** Procédé de préparation de compositions pharmaceutiques, caractérisé en ce que l'on met, à titre de principe actif, l'un au moins des produits de formule (I), tels que définis à la revendication 1, sous une forme destinée à cet usage.

**7.** Procédé de préparation de compositions pharmaceutiques, caractérisé en ce que l'on met, à titre de principe actif, l'un au moins des produits de formule (I), tels que définis à la revendication 2 ou 3, sous une forme destinée à cet usage.

**8.** Procédé de préparation de compositions pharmaceutiques, caractérisé en ce que l'on met, à titre de principe actif, la 1-(styrène sulfonyl) 2-oxo 5-éthoxy pyrrolidine sous une forme destinée à cet usage.

**Claims**
**Claims for the following Contracting States : BE, CH, DE, FR, GB, IT, LI, LU, NL, SE**

**1.** The compounds corresponding to general formula (I):

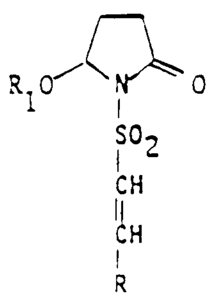

(I)

in which $R_1$ represents a linear or branched alkyl radical containing up to 8 carbon atoms, an acyl radical containing 1 to 6 carbon atoms or a cycloalkyl radical containing 3 to 6 carbon atoms and R represents a phenyl or naphthyl radical, or one of the following radicals: furyl, thienyl, pyranyl, pyridyl, benzofuranyl, isobenzofuranyl, chromanyl, isochromanyl, chromenyl, xanthenyl, phenoxathienyl, oxazolyl, isoxazolyl, furazanyl, phenoxazinyl, thieno[2,3-b]furanyl, 2H-furo[3,2-b]pyranyl, benzoxazolyl or morpholinyl, R being optionally substituted by one or more substituents chosen from the group constituted by hydroxyl, acetoxy, methoxy or benzyloxy radicals, by the radical

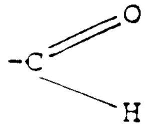 , 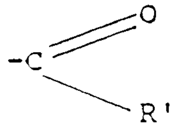 or $-C{=}NOR'$

in which R' represents an alkyl radical containing up to 8 carbon atoms, by methyl, ethyl, propyl or isopropyl radicals, the ethenyl radical or ethynyl radical, fluorine, chlorine, bromine, $SCF_3$, $OCF_3$, $NO_2$, $NH_2$ or C≡N groups and the phenyl radical.

2. The compounds of formula (I) as defined in claim 1, in which R represents a phenyl radical optionally substituted by an alkoxy radical containing up to 4 carbon atoms.

3. The compounds as defined in claim 1 or 2, in which $R_1$ represents an ethyl radical.

4. The compound as defined in claim 3 the name of which follows:
   - 1-(styrene sulphonyl)-2-oxo-5-ethoxy pyrrolidine.

5. As medicaments, the compounds as defined in any one of claims 1 to 3.

6. As a medicament, the compound defined in claim 4.

7. The pharmaceutical compositions containing as active ingredient at least one medicament defined in claim 5 or 6.

8. Preparation process for compounds of formula (I) as defined in any one of claims 1 to 4, characterized in that a compound of formula (II):

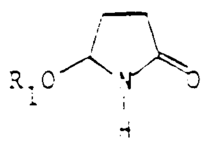

(II)

in which $R_1$ keeps the same meaning as previously, is subjected to the action of a compound of formula (III):

$RCH=CH-SO_2Hal$ (III)

in which Hal represents a chlorine or bromine atom and R keeps the same meaning as in claim 1, in order to obtain the corresponding compound of formula (I).

9. As an intermediate product necessary for implementing the process of claim 8:
   - 4-methoxy styrene sulphonyl chloride.

**Claims for the following Contracting State : AT**

1. Process for preparing compounds corresponding to general formula (I):

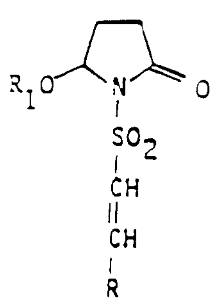

(I)

in which $R_1$ represents a linear or branched alkyl radical containing up to 8 carbon atoms, an acyl radical containing 1 to 6 carbon atoms or a cycloalkyl radical containing 3 to 6 carbon atoms and R represents a phenyl or naphthyl radical, or one of the following radicals: furyl, thienyl, pyranyl, pyridyl, benzofuranyl, isobenzofuranyl, chromanyl, isochromanyl, chromenyl, xanthenyl, phenoxathienyl, oxazolyl, isoxazolyl, furazanyl, phenoxazinyl, thieno[2,3-b]furanyl, 2H-furo[3,2-b]pyranyl, benzoxazolyl or morpholinyl, R being optionally substituted by one or more substituents chosen from the group

constituted by hydroxyl, acetoxy, methoxy or benzyloxy radicals, by the radical

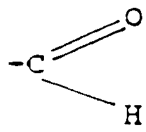 , 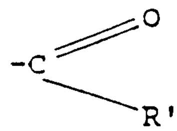 or -C=NOR'

in which R' represents an alkyl radical containing up to 8 carbon atoms, by methyl, ethyl, propyl or isopropyl radicals, the ethenyl radical or ethynyl radical, fluorine, chlorine, bromine, $SCF_3$, $OCF_3$, $NO_2$, $NH_2$ or C≡N groups and the phenyl radical, characterized in that a compound of formula (II):

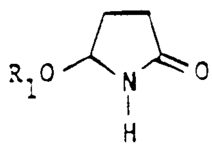

(II)

in which $R_1$ keeps the same meaning as previously, is subjected to the action of a compound of formula (III):

$RCH = CH\text{-}SO_2Hal$ (III)

in which Hal represents a chlorine or bromine atom and R keeps the same meaning as in claim 1, in order to obtain the corresponding compound of formula (I).

2. Process according to claim 1, characterized in that at the start a compound of formula (III) is used in which R represents a phenyl radical optionally substituted by an alkoxy radical containing up to 4 carbon atoms.

3. Process according to claim 1 or 2, characterized in that at the start a compound of formula (II) is used in which $R_1$ represents an ethyl radical.

4. Process according to claim 1, 2 or 3, characterized in that at the start a compound of formula (III) is used in which R represents a phenyl radical and a compound of formula (II) is used in which $R_1$ represents an ethyl radical.

**Claims for the following Contracting State : ES**

1. Process for preparing compounds corresponding to general formula (I):

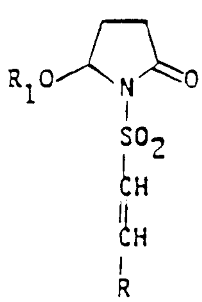

(I)

in which $R_1$ represents a linear or branched alkyl radical containing up to 8 carbon atoms, an acyl radical containing 1 to 6 carbon atoms or a cycloalkyl radical containing 3 to 6 carbon atoms and R represents a phenyl or naphthyl radical, or one of the following radicals: furyl, thienyl, pyranyl, pyridyl,

benzofuranyl, isobenzofuranyl, chromanyl, isochromanyl, chromenyl, xanthenyl, phenoxathienyl, oxazolyl, isoxazolyl, furazanyl, phenoxazinyl, thieno[2,3-b]furanyl, 2H-furo[3,2-b]pyranyl, benzoxazolyl or morpholinyl, R being optionally substituted by one or more substituents chosen from the group constituted by hydroxyl, acetoxy, methoxy or benzyloxy radicals, by the radical

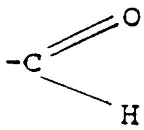 , 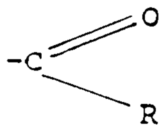 or $-C{=}NOR'$

in which R' represents an alkyl radical containing up to 8 carbon atoms, by methyl, ethyl, propyl or isopropyl radicals, the ethenyl radical or ethynyl radical, fluorine, chlorine, bromine, $SCF_3$, $OCF_3$, $NO_2$, $NH_2$ or C≡N groups and the phenyl radical, characterized in that a compound of formula (II):

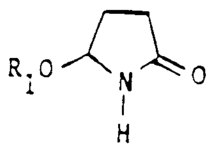

(II)

in which $R_1$ keeps the same meaning as previously, is subjected to the action of a compound of formula (III):

$RCH{=}CH\text{-}SO_2Hal$ (III)

in which Hal represents a chlorine or bromine atom and R keeps the same meaning as in claim 1, in order to obtain the corresponding compound of formula (I).

2. Process according to claim 1, characterized in that at the start a compound of formula (III) is used in which R represents a phenyl radical optionally substituted by an alkoxy radical containing up to 4 carbon atoms.

3. Process according to claim 1 or 2, characterized in that at the start a compound of formula (II) is used in which $R_1$ represents an ethyl radical.

4. Process according to claim 1, 2 or 3, characterized in that at the start a compound of formula (III) is used in which R represents a phenyl radical and a compound of formula (II) is used in which $R_1$ represents an ethyl radical.

**Claims for the following Contracting State: GR**

1. Process for preparing compounds corresponding to general formula (I):

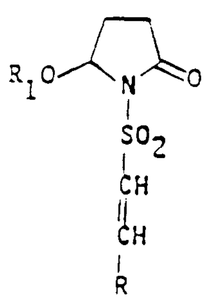

(I)

in which $R_1$ represents a linear or branched alkyl radical containing up to 8 carbon atoms, an acyl radical containing 1 to 6 carbon atoms or a cycloalkyl radical containing 3 to 6 carbon atoms and R represents a phenyl or naphthyl radical, or one of the following radicals: furyl, thienyl, pyranyl, pyridyl, benzofuranyl, isobenzofuranyl, chromanyl, isochromanyl, chromenyl, xanthenyl, phenoxathienyl, oxazolyl, isoxazolyl, furazanyl, phenoxazinyl, thieno[2,3-b]furanyl, 2H-furo[3,2-b]pyranyl, benzoxazolyl or morpholinyl, R being optionally substituted by one or more substituents chosen from the group constituted by hydroxyl, acetoxy, methoxy or benzyloxy radicals, by the radical

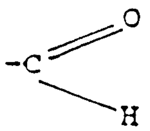

,

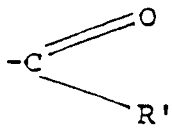

or $-C=NOR'$

in which R' represents an alkyl radical containing up to 8 carbon atoms, by methyl, ethyl, propyl or isopropyl radicals, the ethenyl radical or ethynyl radical, fluorine, chlorine, bromine, $SCF_3$, $OCF_3$, $NO_2$, $NH_2$ or C≡N groups and the phenyl radical, characterized in that a compound of formula (II):

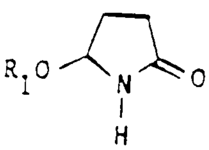

(II)

in which $R_1$ keeps the same meaning as previously, is subjected to the action of a compound of formula (III):

$RCH=CH\text{-}SO_2Hal$ (III)

in which Hal represents a chlorine or bromine atom and R keeps the same meaning as in claim 1, in order to obtain the corresponding compound of formula (I).

**2.** Process according to claim 1, characterized in that at the start a compound of formula (III) is used in which R represents a phenyl radical optionally substituted by an alkoxy radical containing up to 4 carbon atoms.

**3.** Process according to claim 1 or 2, characterized in that at the start a compound of formula (II) is used in which $R_1$ represents an ethyl radical.

**4.** Process according to claim 1, 2 or 3, characterized in that at the start a compound of formula (III) is used in which R represents a phenyl radical and a compound of formula (II) is used in which $R_1$ represents an ethyl radical.

**5.** As a new intermediate product necessary for implementing the process of claim 1:
- 4-methoxy styrene sulphonyl chloride.

**6.** Preparation process for pharmaceutical compositions, characterized in that at least one of the products of formula (I), as defined in claim 1, is used as active ingredient in a form intended for this use.

**7.** Preparation process for pharmaceutical compositions, characterized in that at least one of the products of formula (I), as defined in claim 2 or 3, is used as active ingredient in a form intended for this use.

**8.** Preparation process for pharmaceutical compositions, characterized in that 1-(styrene sulphonyl)-2-oxo-5-ethoxy pyrrolidine is used as active ingredient in a form intended for this use.

**Patentansprüche**

**Patentansprüche für folgende Vertragsstaaten : BE, CH, DE, FR, GB, IT, LI, LU, NL, SE**

1. Verbindungen der allgemeinen Formel (I)

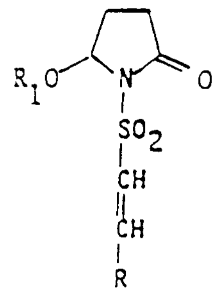

(I)

worin $R_1$ für einen linearen oder verzweigten Alkylrest mit bis zu 8 Kohlenstoffatomen, einen Acylrest mit 1 bis 6 Kohlenstoffatomen oder einen Cycloalkylrest mit 3 bis 6 Kohlenstoffatomen steht und R einen Phenyl- oder Naphthylrest oder einen Furyl-, Thienyl-, Pyranyl-, Pyridyl-, Benzofuranyl-, Isobenzofuranyl-, Chromanyl-, Isochromanyl-, Chromenyl-, Xanthenyl-, Phenoxathienyl-, Oxazolyl-, Isoxazolyl-, Furazanyl-, Phenoxazinyl-, Thieno[2,3-b]furanyl-, 2H-Furo[3,2-b]pyranyl-, Benzoxazolyl- oder Morpholinylrest bedeutet, wobei R gegebenenfalls substituiert sein kann durch einen oder mehrere Substituenten, ausgewählt unter den Hydroxyl-, Acetoxy-, Methoxy- oder Benzyloxyresten, unter dem Rest

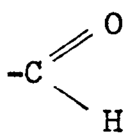 , 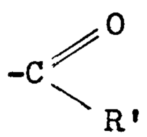 oder $-C=NOR'$ ,

worin R' für einen Alkylrest mit bis zu 8 Kohlenstoffatomen steht, unter den Methyl-, Ethyl-, Propyl- oder Isopropylresten, dem Ethenylrest oder dem Ethinylrest, Fluor, Chlor, Brom, den Gruppen $SCF_3$, $OCF_3$, $NO_2$, $NH_2$ oder C≡N und dem Phenylrest.

2. Verbindungen der Formel (I) gemäß Anspruch 1, worin R für einen Phenylrest steht, der gegebenenfalls durch einen Alkoxyrest mit bis zu 4 Kohlenstoffatomen substituiert ist.

3. Verbindungen gemäß Anspruch 1 oder 2, worin $R_1$ einen Ethylrest bedeutet.

4. Verbindung gemäß Anspruch 3 mit der folgenden Bezeichnung:
   1-(Styrolsulfonyl)-2-oxo-5-ethoyxypyrrolidin.

5. Als Arzneimittel die Verbindungen gemäß einem der Ansprüche 1 bis 3.

6. Als Arzneimittel die Verbindung gemäß Anspruch 4.

7. Pharmazeutische Zusammensetzungen, enthaltend als Wirkstoff zumindest ein Arzneimittel gemäß Anspruch 5 oder 6.

8. Verfahren zur Herstellung der Verbindungen der Formel (I) gemäß einem der Ansprüche 1 bis 4, dadurch gekennzeichnet, daß man eine Verbindung der Formel (II)

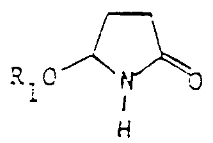

(II)

worin $R_1$ die vorstehend angegebene Bedeutung besitzt, der Einwirkung einer Verbindung der Formel (III)

$RCH = CH\text{-}SO_2Hal$ (III)

unterzieht, worin Hal für ein Chlor- oder Bromatom steht und R die in Anspruch 1 angegebene Bedeutung besitzt, um die entsprechende Verbindung der Formel (I) zu erhalten.

**9.** Als für die Durchführung des Verfahrens gemäß Anspruch 8 erforderliches Zwischenprodukt: das 4-Methoxystyrolsulfonylchlorid.

**Patentansprüche für folgenden Vertragsstaat : AT**

**1.** Verfahren zur Herstellung von Verbindungen der allgemeinen Formel (I)

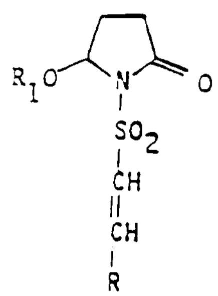

(I)

worin $R_1$ für einen linearen oder verzweigten Alkylrest mit bis zu 8 Kohlenstoffatomen, einen Acylrest mit 1 bis 6 Kohlenstoffatomen oder einen Cycloalkylrest mit 3 bis 6 Kohlenstoffatomen steht und R einen Phenyl- oder Naphthylrest oder einen Furyl-, Thienyl-, Pyranyl-, Pyridyl-, Benzofuranyl-, Isobenzofuranyl-, Chromanyl-, Isochromanyl-, Chromenyl-, Xanthenyl-, Phenoxathienyl, Oxazolyl-, Isoxazolyl-, Furazanyl-, Phenoxazinyl-, Thieno[2,3-b]furanyl-, 2H-Furo[3,2-b]pyranyl-, Benzoxazolyl- oder Morpholinylrest bedeutet, wobei R gegebenenfalls substituiert sein kann durch einen oder mehrere Substituenten, ausgewählt unter den Hydroxyl-, Acetoxy-, Methoxy- oder Benzyloxyresten, unter dem Rest

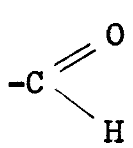 , 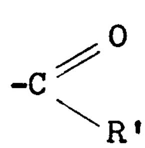 oder -C=NOR',

worin R' für einen Alkylrest mit bis zu 8 Kohlenstoffatomen steht, unter den Methyl-, Ethyl, Propyl- oder Isopropylresten, dem Ethenylrest oder dem Ethinylreste, Fluor, Chlor, Brom, den Gruppen $SCF_3$, $OCF_3$, $NO_2$, NH2 oder C≡N und dem Phenylrest, dadurch **gekennzeichnet,** daß man eine Verbindung der Formel (II)

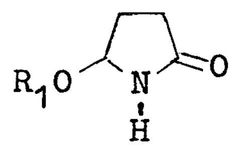

(II)

worin $R_1$ die vorstehend angegebene Bedeutung besitzt, der Einwirkung einer Verbindung der Formel (III)

$RCH=CH\text{-}SO_2Hal$ (III)

unterzieht, worin Hal ein Chlor- oder Bromatom bedeutet und R die in Anspruch 1 angegebene Bedeutung besitzt, um die entsprechende Verbindung der Formel (I) zu erhalten.

**2.** Verfahren gemäß Anspruch 1, dadurch gekennzeichnet, daß man von einer Verbindung der Formel (III) ausgeht, worin R einen Phenylrest bedeutet, der gegebenenfalls durch einen Alkoxyrest mit bis zu 4 Kohlenstoffatomen substituiert ist.

**3.** Verfahren gemäß Anspruch 1 oder 2, dadurch gekennzeichnet, daß man von einer Verbindung der Formel (II) ausgeht, worin $R_1$ für einen Ethylrest steht.

**4.** Verfahren gemäß Anspruch 1, 2 oder 3, dadurch gekennzeichnet, daß man von einer Verbindung der Formel (III), worin R für einen Phenylrest steht, und einer Verbindung der Formel (II), worin $R_1$ einen Ethylrest bedeutet, ausgeht.

**Patentansprüche für folgenden Vertragsstaat : ES**

**1.** Verfahren zur Herstellung von Verbindungen der allgemeinen Formel (I)

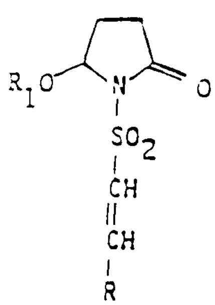

(I)

worin $R_1$ für einen linearen oder verzweigten Alkylrest mit bis zu 8 Kohlenstoffatomen, einen Acylrest mit 1 bis 6 Kohlenstoffatomen oder einen Cycloalkylrest mit 3 bis 6 Kohlenstoffatomen steht und R einen Phenyl- oder Naphthylrest oder einen Furyl-, Thienyl-, Pyranyl-, Pyridyl-, Benzofuranyl-, Isobenzofuranyl-, Chromanyl-, Isochromanyl-, Chromenyl-, Xanthenyl-, Phenoxathienyl-, Oxazolyl-, Isoxazolyl-, Furazanyl-, Phenoxazinyl-, Thieno[2,3-b]furanyl-, 2H-Furo[3,2-b]pyranyl-, Benzoxazolyl- oder Morpholinylrest bedeutet, wobei R gegebenenfalls substituiert sein kann durch einen oder mehrere Substituenten, ausgewählt unter den Hydroxyl-, Acetoxy-, Methoxy- oder Benzyloxyresten, unter dem Rest

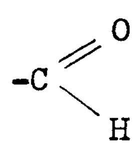 , 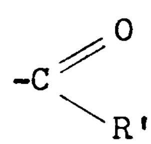 oder $-C=NOR'$,

worin R' für einen Alkylrest mit bis zu 8 Kohlenstoffatomen steht, unter den Methyl-, Ethyl-, Propyl-

oder Isopropylresten, dem Ethenylrest oder dem Ethinylrest , Fluor, Chlor, Brom, den Gruppen $SCF_3$, $OCF_3$, $NO_2$, $NH_2$ oder C≡N und dem Phenylrest, dadurch **gekennzeichnet,** daß man eine Verbindung der Formel (II)

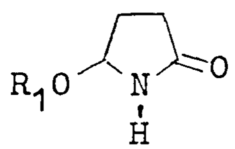

(II)

worin $R_1$ die vorstehend angegebene Bedeutung besitzt, der Einwirkung einer Verbindung der Formel (III)

$RCH = CH\text{-}SO_2Hal$ (III)

unterzieht, worin Hal ein Chlor- oder Bromatom bedeutet und R die in Anspruch 1 angegebene Bedeutung besitzt, um die entsprechende Verbindung der Formel (I) zu erhalten.

**2.** Verfahren gemäß Anspruch 1, dadurch gekennzeichnet, daß man von einer Verbindung der Formel (III) ausgeht, worin R einen Phenylrest bedeutet, der gegebenenfalls durch einen Alkoxyrest mit bis zu 4 Kohlenstoffatomen substituiert ist.

**3.** Verfahren gemäß Anspruch 1 oder 2, dadurch gekennzeichnet, daß man von einer Verbindung der Formel (II) ausgeht, worin $R_1$ für einen Ethylrest steht.

**4.** Verfahren gemäß Anspruch 1, 2 oder 3, dadurch gekennzeichnet, daß man von einer Verbindung der Formel (III), worin R für einen Phenylrest steht, und einer Verbindung der Formel (II), worin $R_1$ einen Ethylrest bedeutet, ausgeht.

**Patentansprüche für folgenden Vertragsstaat : GR**

**1.** Verfahren zur Herstellung von Verbindungen der allgemeinen Formel (I)

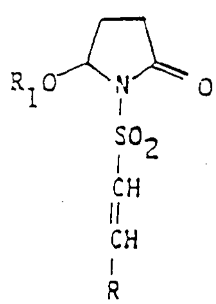

(I)

worin $R_1$ für einen linearen oder verzweigten Alkylrest mit bis zu 8 Kohlenstoffatomen, einen Acylrest mit 1 bis 6 Kohlenstoffatomen oder einen Cycloalkylrest mit 3 bis 6 Kohlenstoffatomen steht und R einen Phenyl- oder Naphthylrest oder einen Furyl-, Thienyl-, Pyranyl-, Pyridyl-, Benzofuranyl-, Isobenzofuranyl-, Chromanyl-, Isochromanyl-, Chromenyl-, Xanthenyl-, Phenoxathienyl-, Oxazolyl-, Isoxazolyl-, Furazanyl-, Phenoxazinyl-, Thieno[2,3-b]furanyl-, 2H-Furo[3,2-b]pyranyl-, Benzoxazolyl- oder Morpholinylrest bedeutet, wobei R gegebenenfalls substituiert sein kann durch einen oder mehrere Substituenten, ausgewählt unter den Hydroxyl-, Acetoxy-, Methoxy- oder Benzyloxyresten, unter dem Rest

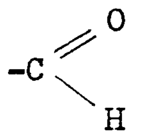 , 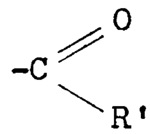 oder -C=NOR',

worin R' für einen Alkylrest mit bis zu 8 Kohlenstoffatomen steht, unter den Methyl-, Ethyl-, Propyl- oder Isopropylresten, dem Ethenylrest oder dem Ethinylrest , Fluor, Chlor, Brom, den Gruppen $SCF_3$, $OCF_3$, $NO_2$, NH2 oder C≡N und dem Phenylrest, dadurch **gekennzeichnet,** daß man eine Verbindung der Formel (II)

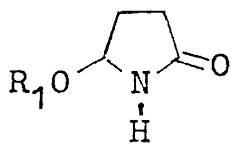

(II)

worin $R_1$ die vorstehend angegebene Bedeutung besitzt, der Einwirkung einer Verbindung der Formel (III)

$RCH = CH\text{-}SO_2Hal$ (III)

unterzieht, worin Hal ein Chlor- oder Bromatom bedeutet und R die in Anspruch 1 angegebene Bedeutung besitzt, um die entsprechende Verbindung der Formel (I) zu erhalten.

**2.** Verfahren gemäß Anspruch 1, dadurch gekennzeichnet, daß man von einer Verbindung der Formel (III) ausgeht, worin R einen Phenylrest bedeutet, der gegebenenfalls durch einen Alkoxyrest mit bis zu 4 Kohlenstoffatomen substituiert ist.

**3.** Verfahren gemäß Anspruch 1 oder 2, dadurch gekennzeichnet, daß man von einer Verbindung der Formel (II) ausgeht, worin $R_1$ für einen Ethylrest steht.

**4.** Verfahren gemäß Anspruch 1, 2 oder 3, dadurch gekennzeichnet, daß man von einer Verbindung der Formel (III), worin R für einen Phenylrest steht, und einer Verbindung der Formel (II), worin $R_1$ einen Ethylrest bedeutet, ausgeht.

**5.** Als für die Durchführung des Verfahrens gemäß Anspruch 1 erforderliches Zwischenprodukt:
das 4-Methoxystyrolsulfonylchlorid.

**6.** Verfahren zur Herstellung von pharmazeutischen Zusammensetzungen, dadurch gekennzeichnet, daß man als Wirkstoff zumindest eines der Produkte der Formel (I), wie in Anspruch 1 definiert, in eine für diese Verwendung bestimmte Form bringt.

**7.** Verfahren zur Herstellung von pharmazeutischen Zusammensetzungen, dadurch gekennzeichnet, daß man als Wirkstoff zumindest eines der Produkte der Formel (I), wie in Anspruch 2 oder 3 definiert, in eine für diese Verwendung bestimmte Form bringt.

**8.** Verfahren zur Herstellung von pharmazeutischen Zusammensetzungen, dadurch gekennzeichnet, daß man als Wirkstoff das 1-(Styrolsulfonyl)-2-oxo-5-ethoxypyrrolidin in eine für diese Verwendung bestimmte Form bringt.